(19) Europäisches Patentamt
European Patent Office
Office européen des brevets

(11) Numéro de publication: **0 021 991**
**B1**

# (12) FASCICULE DE BREVET EUROPEEN

(45) Date de publication du fascicule de brevet: **05.05.82**

(51) Int. Cl.³: **C 07 C 149/24,** A 61 K 31/13

(21) Numéro de dépôt: **80400894.4**

(22) Date de dépôt: **18.06.80**

(54) **Dérivés de la cystamine, leur préparation et compositions pharmaceutiques les contenant.**

(30) Priorité: **19.06.79 FR 7915672**

(43) Date de publication de la demande:
**07.01.81 Bulletin 81/1**

(45) Mention de la délivrance du brevet:
**05.05.82 Bulletin 82/18**

(84) Etats contractants désignés:
**BE CH DE FR GB IT LI**

(56) Documents cités:
**CHEMICAL ABSTRACTS, vol. 85, n° 21, 22 novembre 1976, réf. 159 373a, page 490 Columbus, Ohio, US G. EISENBRAND et al.: "Some new congeners of the anticancer agent 1, 3-bis(2-chloroethyl)-1-nitrosourea (BCNU). Synthesis of preliminary evaluation of their chemotherapeutic potential"**

**CHEMICAL ABSTRACTS, vol. 87, n° 23, 5 décembre 1977, réf. 183 955d, page 563 Columbus, Ohio, US I. BARACU et al.: "Potential anticancer agents. XIII. Synthesis of new aliphatic and cycloaliphatic N-nitrosoureas"**

(73) Titulaire: **ANVAR Agence Nationale de Valorisation de la Recherche, 13, rue Madeleine Michelis, F-92522 Neuilly-sur-Seine (FR)**

(72) Inventeur: **Oiry, Joel, 31, Lotissement Châteaubon Route de Laverune, F34000 Montpellier (FR)**
Inventeur: **Imbach, Jean-Louis, Prof., Chemin du Clos des Oliviers 1108 rue de las Sordes, F-34000 Montpellier (FR)**

(74) Mandataire: **Corre, Jacques et al, Cabinet Regimbeau 26, Avenue Kléber, F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 021 991 B1

ACTORUM AG.

# Dérivés de la cystamine, leur préparation et compositions pharmaceutiques les contenant.

La présente invention concerne de nouveaux composés chimiques utiles comme médicaments ainsi que leur procédé de préparation.

L'invention concerne des composés de formule:

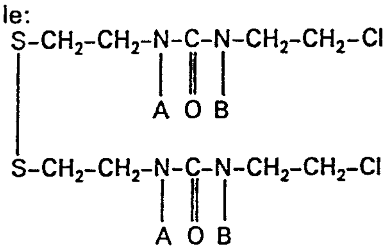

(I)

dans laquelle l'un des substituants A et B est l'hydrogène et l'autre le radical -N=O.

Ces composés peuvent être préparés par nitrosation du composé de formule:

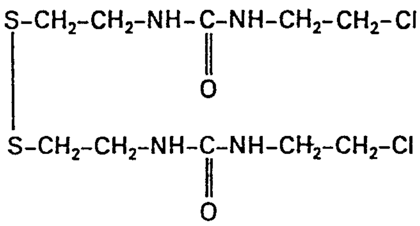

(II)

La nitrosation du composé de formule II peut être effectuée notamment à l'aide du chlorure de nitrosyle ou du nitrite de sodium, en particulier en utilisant le chlorure de nitrosyle en solution pyridinique, par exemple à une température comprise entre −5 et +5°C, de préférence 0°C, ou bien par le nitrite de sodium dans l'acide formique pur ou dans l'acide formique aqueux à 50%, on peut également utiliser le chlorure de nitrosyle dans le dichlorométhane, de préférence à une température inférieure à −10°C.

Le composé de formule II peut être préparé par condensation de la cystamine avec l'isocyanate de chloro-2-éthyle, la cystamine étant de préférence préparée in situ par action de la triéthylamine sur le chlorhydrate de cystamine.

Le schéma, ci-après, résume la synthèse des composés selon la présente invention.

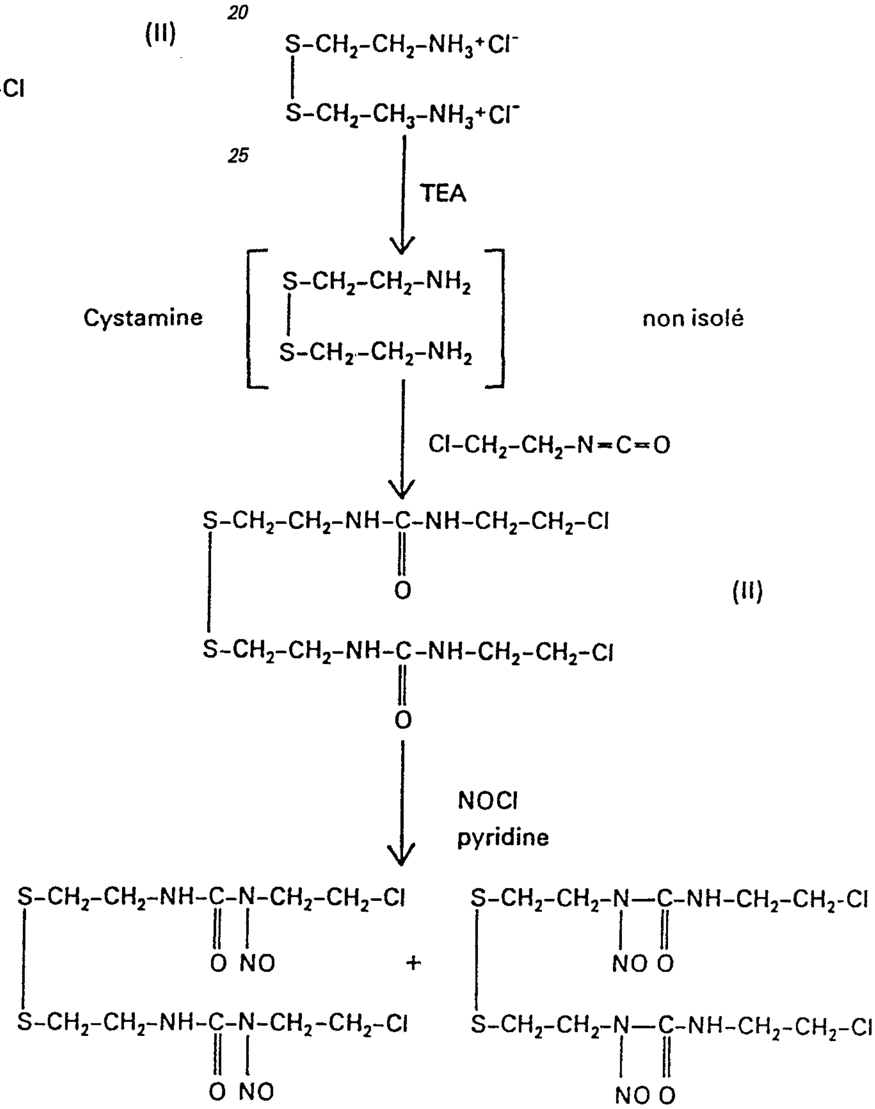


(II)

(IA) Rendement: 62%

(IB) Rendement: 38%

Les composés selon la présente invention se sont révélés particulièrement efficaces dans le traitement de certaines tumeurs, en particulier dans le traitement de la leucémie L 1210.

La présente invention concerne donc également, à titre de médicaments nouveaux, les composés selon la présente invention ainsi que des compositions pharmaceutiques comprenant, à titre de principe actif, au moins un composé selon la présente invention.

Les compositions pharmaceutiques selon la présente invention sont plus particulièrement des compositions utilisables par voie orale, en particulier sous forme de gélule, compte tenu de leur faible solubilité dans l'eau.

Bien entendu, les dosages journaliers appliqués peuvent varier en fonction de la tumeur et de l'état général du patient et peuvent être, en général, compris entre 1 et 5 mg/kg par jour en mono-injection ou en poly-injections.

Les exemples suivants sont destinés à illustrer la préparation des composés selon la présente invention.

Exemple

Préparation de la di-[(chloro-2-éthyl)-2-carbamoyl]-N,N-cystamine (II)

Une solution de 50 g (0,22 mole) de chlorhydrate de cystamine dans 200 $cm^3$ d'eau distillée est neutralisée à 0°C sous agitation avec 61 ml (0,44 mole) de triéthylamine (TEA) fraîchement distillée.

Après l'addition de la TEA, et en maintenant la température réactionnelle à 0°C, on verse goutte à goutte 40,3 ml (0,47 mole) d'isocyanate de chloro-2-éthyle. L'addition du réactif s'effectue en 2 heures, ce qui laisse déposer une poudre blanche. Au terme des 2 heures, on laisse revenir le mélange à température ambiante en maintenant l'agitation durant une nuit.

Le précipité est essoré et lavé avec 5×100 $cm^3$ d'eau distillée glacée, puis avec 3×100 $cm^3$ d'un mélange de méthanol et d'éther (60–120) et enfin avec 2×250 $cm^3$ d'éther.

Après séchage sous vide dans un dessicateur sur anhydride phosphorique, on recueille 78 g du composé II qui cristallise du méthanol en plaquettes incolores.

F = 144–145°C.

CCM = Rf: 0,75 (éluant: chloroforme-méthanol 8–2).

Analyse $C_{10}H_{20}S_2N_4O_2Cl_2$

| | | | |
|---|---|---|---|
| calculé (%): | C 33,05 | H 5,50 | N 15,42 |
| trouvé (%): | C 32,96 | H 5,96 | N 15,26. |

Spectre IR (KBr) $\nu$ $cm^{-1}$
(NH) 3330; (CH)2960,2920; (C=O)1625;
(Amide)1570,1520.

Spectre RMN (DMSOd6)
NH: (t) centré à 6,28 ppm, 4 H échangeables à $D_2O$
$CH_2Cl$: (t) centré à 3,56 ppm, 4H
$N\text{-}CH_2$: (m) centré à 3,6 ppm, 8 H
$S\text{-}CH_2$: (t) centré à 2,73 ppm, 4 H

Préparation de la di-[(chloro-2-éthyl)-2-N-nitroso-N-carbamoyl]-N,N-cystamine (IA)

Une suspension agitée de 20 g de composé II (0,055 mole) dans 150 $cm^3$ de pyridine anhydre est traitée à 0°C par barbottage de chlorure de nitrosyle. Lorsqu'une solution homogène est obtenue et lorsqu'une coloration orangée persiste, on arrête le dégagement de chlorure en maintenant l'agitation à 0°C durant 1 heure.

On laisse ensuite revenir à température ambiante la solution qui est ensuite versée sur 1000 $cm^3$ d'eau distillée glacée, ce qui provoque la formation d'une huile épaisse orangée.

Le mélange est filtré sur 150 g de célite et lavé à l'eau distillée afin d'éliminer la solution de l'huile qui est retenue par le support.

Après différents lavages de la célite par de l'acétate d'éthyle (4×100 $cm^3$) on réunit les phases organiques et on les lave à l'eau distillée (3×100 $cm^3$) on sèche sur sulfate de sodium et on évapore à sec sous vide (température du bain-marie inférieure à 40°C). On recueille une huile orangée qui cristallise de l'éther. Rendement: 96%.

Une purification par filtration sur 200 g de silicagel (kieselgel 60 = 70–230 mesh) avec de l'acétate d'éthyle comme éluant est nécessaire. On cristallise un mélange d'acétate d'éthyle-éther 1–9.

F = 78–79°C. CCM = 1 spot dans quatre systèmes d'éluants différents et également 1 spot après plusieurs migrations dans les deux dimensions.

Analyse $C_{10}H_{18}S_2N_6O_4Cl_2$

| | | | |
|---|---|---|---|
| calculé (%): | C 28,50 | H 4,27 | N 19,95 |
| trouvé (%): | C 28,39 | H 4,27 | N 20,04 |

Spectre IR (Kbr) $\nu$ $cm^{-1}$
(NH)3340; (CH)2960,2940; (C=0)1698;
(Amide)1530; (N-NO)1480.

Spectre RMN (DMSOd6)
NH: (t) centré à 8,92 ppm, 2 H échangeables à $D_2O$
$CH_2Cl$: (t) centré à 4,02 ppm, 4 H
$N\text{-}CH_2$: (m) centré à 3,64 ppm, 8 H
$S\text{-}CH_2$: (t) centré à 2,73 ppm, 4 H.

Dans le même spectre on peut observer d'autres signaux correspondant au composé IB: la di-[(chloro-2-éthyl)-2-N-carbamoyl]-N,N-nitroso-N,N-cystamine.

Les deux protons NH sont confondus avec ceux du composé IA ainsi que ceux des $CH_2Cl$ qui apparaissent avec le triplet de IA.

Les protons $N\text{-}CH_2$ qui sortent avec ceux de leur isomère IA (m), mais avec une intensité plus faible, expliquent la complexité du signal.

Seuls les $S-CH_2$ sont bien distincts de l'autre isomère; ils forment également un triplet qui est centré à 2,93 ppm au lieu de 2,73 ppm. En faisant le rapport d'intégration des protons $S-CH_2$, les deux isomères de positions sont dans une proportion de 62% pour le composé IA et 38% pour le composé IB.

Les points de fusion non corrigés ont été pris en capillaire sur un appareil Gallenkamp.

Les chromatographies sur couche mince ont été réalisées sur feuilles d'aluminium recouvertes de gel de silice 60F254 Merck®. Les spots sont détectés à l'UV ou révélés en atmosphère d'iode, ou par pulvérisation de ninhydrine, suivie du chauffage de la plaque.

Les spectres IR ont été enregistrés sur un spectrographe Beckman IR4.

Les spectres de RMN du $^1H$ ont été enregistrés sur un spectrographe HA-100 avec le TMS comme référence interne. (d = doublet, t = triplet, m = multiplet).

Les composés selon la présente invention ont été étudiés sur diverses tumeurs en comparaison avec d'autres nitrosourées présentant également des propriétés oncostatiques:

- BCNU: (1,3-bis-2-chloroethyl)-1 nitrosourée;

- CCNU: (chloro-2-éthyl)-1 cyclohexyl-3 nitrosourée;

- MeCCNU: (chloro-2-éthyl)-1 (methyl-4 cyclohexyl)-3;

- RFCNU: (chloro-2-éthyl)-1 ribofuranosyl-isopropylidène-2'-3' paranitrobenzoate-5')-3' nitrosourée;

- RPCNU: (chloro-2-éthyl)-1 ribopyranosyl triacétate-2',3',4')-3 nitrosourée;

- Chlorozotocine.

Ces composés sont décrits notamment dans:

- J.L. Imbach et col., Biomedicine Express, 1975, 23, 410–13;

- J.L. Montero et col., Eur. J. Med. Chem., 1976, 11, 183;

- J.L. Montero et col., C.R. Acad. Sc. Paris, Série C, 1974, t 279, 809;

- J.L. Montero et col., Eur. J. Med. Chem., 1978, 13, 421.

Les résultats sont donnés dans le tableau suivant et dans la figure annexée qui donnent:
T/C (%) (durée de survie moyenne des souris traitées/durée de survie moyenne des souris témoins - 100)
∞: plus de 50% des animaux traités sont guéris.

Il ressort de ce tableau et de la figure annexée que les produits selon l'invention présentent une remarquable activité oncostatique en particulier sur L 1210, Lewis Lung Tumor et Colon 26.

En outre, la $DL_{50}$ aiguë est de 70 mg/kg par voie intrapéritonéale, c'est-à-dire plus avantageuse que pour les autres nitrosourées mentionnées.

**Revendications**

1. Composé de formule:

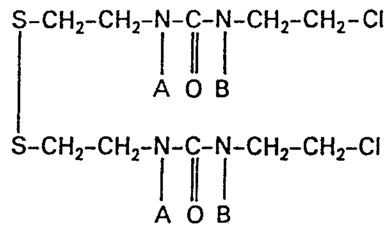

(I)

dans laquelle l'un des substituants A et B est l'hydrogène et l'autre le radical -N=O.

2. Un composé selon la revendication 1 de formule:

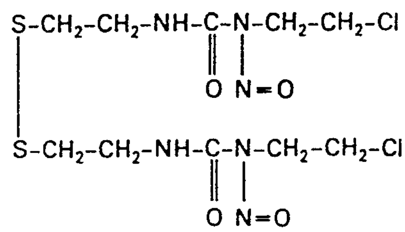

3. Procédé de préparation des composés selon l'une des revendications 1 et 2, caractérisé en ce que l'on effectue la nitrosation du composé de formule:

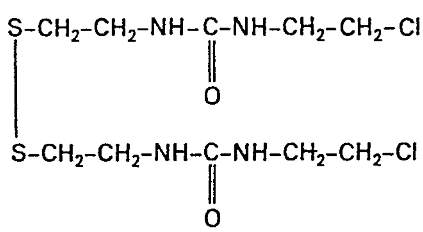

(II)

4. Procédé selon la revendication 3, caractérisé en ce que la nitrosation est effectuée par le chlorure de nitrosyle ou le nitrite de sodium.

5. Procédé selon la revendication 4, caractérisé en ce que la nitrosation est effectuée par le chlorure de nitrosyle en solution pyridinique, par le nitrite de sodium dans l'acide formique, par le chlorure de nitrosyle dans le dichlorométhane.

6. Procédé selon la revendication 4, caractérisé en ce que la nitrosation est effectuée par le chlorure de nitrosyle en solution pyridinique à une température comprise entre −5 et +5°C.

7. Procédé selon l'une des revendications 3 à 6, caractérisé en ce que le composé de formule II est préparé par condensation de la cystamine avec l'isocyanate de chloro-2-éthyle.

8. Procédé delon la revendication 7, caractérisé en ce que la cystamine est préparée in situ par action de la triéthylamine sur le chlorhydrate de cystamine.

9. Composition pharmaceutique caractérisée

en ce qu'elle comporte à titre de principe actif au moins un composé selon les revendications 1 et 2.

10. Composition pharmaceutique selon la revendication 9, caractérisée en ce qu'elle se présente sous une forme applicable par voie orale.

**Patentansprüche**

1. Verbindung der Formel

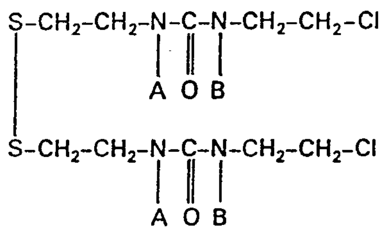

in der einer der Substituenten A oder B Wasserstoff und der andere der Rest -N=O ist.

2. Verbindung nach Anspruch 1 gemäss der Formel

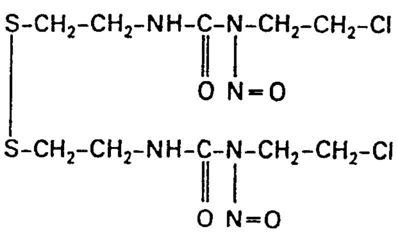

3. Verfahren zur Herstellung der Verbindungen gemäss einem der Ansprüche 1 und 2, dadurch gekennzeichnet, dass man die Verbindung gemäss Formel

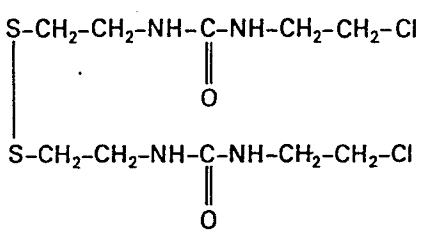

nitrosiert.

4. Verfahren nach Anspruch 3, dadurch gekennzeichnet, dass die Nitrosierung mit Nitrosylchlorid oder Natriumnitrit durchgeführt wird.

5. Verfahren nach Anspruch 4, dadurch gekennzeichnet, dass die Nitrosierung mit Nitrosylchlorid in Pyridinlösung, mit Natriumnitrit in Ameisensäure, mit Nitrosylchlorid in Dichlormethan durchgeführt wird.

6. Verfahren nach Anspruch 4, dadurch gekennzeichnet, dass die Nitrosierung mit Nitrosylchlorid in Pyridinlösung bei einer Temperatur im Bereich von −5 bis +5°C durchgeführt wird.

7. Verfahren nach einem der Ansprüche 3 bis 6, dadurch gekennzeichnet, dass die Verbindung der Formel II durch Kondensation von Cystamin mit 2-Chloräthylisocyanat hergestellt wird.

8. Verfahren nach Anspruch 7, dadurch gekennzeichnet, dass das Cystamin in situ durch Einwirkung von Triäthylamin auf das Cystaminchlorhydrat hergestellt wird.

9. Pharmazeutische Zusammensetzung, gekennzeichnet durch Gehalt an mindestens einer Verbindung gemäss Ansprüchen 1 und 2 als Wirkstoffkomponente.

10. Pharmazeutische Zusammensetzung nach Anspruch 9, dadurch gekennzeichnet, dass sie in oral verabreichbarer Form vorliegt.

**Claims**

1. Compounds corresponding to the following formula

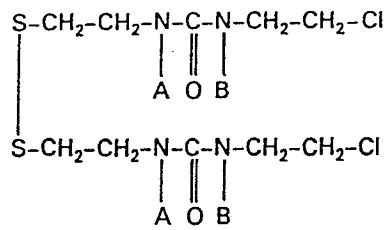

(I)

in which one of the substituents A and B is hydrogen and the other the radical -N=O.

2. Compound according to claim 1 corresponding to the formula

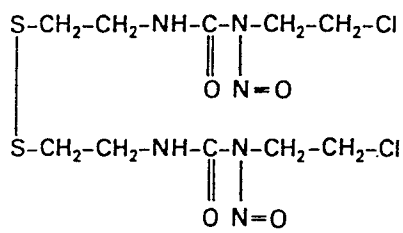

3. Process for preparing the compounds according to anyone of claim 1 or 2, wherein nitroso groups are introduced into a compound corresponding to the following formula

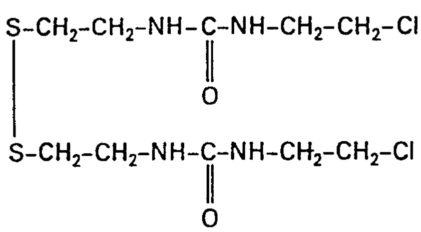

(II)

4. Process according to claim 3, wherein said nitroso groups are introduced using a compound selected from nitrosyl chloride and sodium nitrite.

5. Process according to claim 4, wherein said nitroso group is introduced by nitrosyl chloride in solution in pyridine; sodium nitrite in formic acid and nitrosyl chloride in dichloromethane.

6. Process according to claim 4, wherein said nitroso group is introduced by nitrosyl chloride in solution in pyridine at a temperature in the range from about −5 to about +5°C.

7. Process according to claim 3 wherein said compound of formula II is prepared by condensing cystamine with 2-chloroethyl isocyanate.

8. Process according to claim 7 wherein said cystamine is prepared in situ by reacting triethyl amine with cystamine hydrochloride.

9. Pharmaceutical composition, which contains at least one compound according to anyone of claim 1 and 2 as active ingredient.

10. Pharmaceutical composition according to claim 10, which is made up in a form suitable for oral administration.

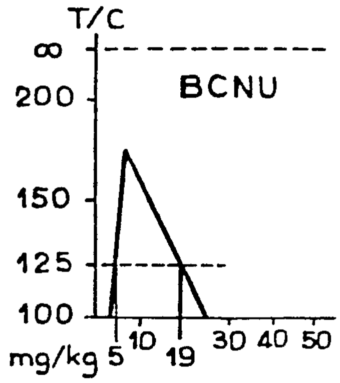


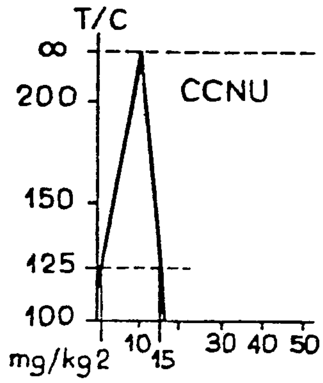


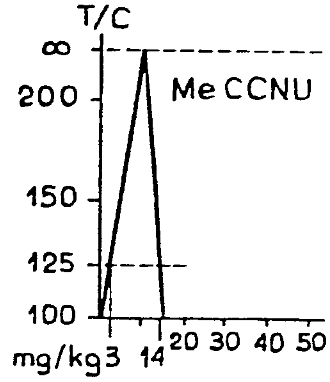


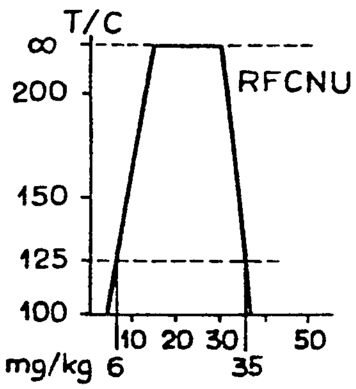


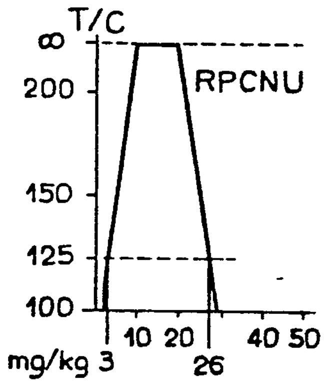


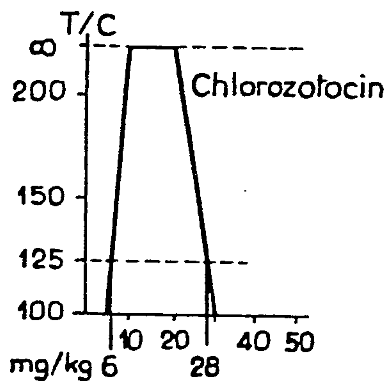


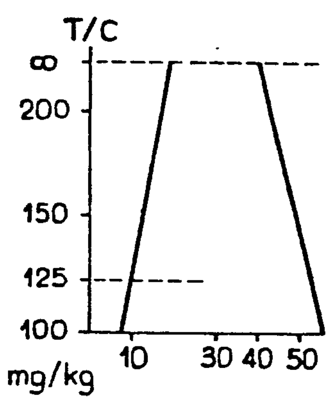


Mélange de
62 % de IA
38 % de IB